(19) Europäisches Patentamt
European Patent Office
Office européen des brevets

(11) EP 2 198 831 B1

(12) FASCICULE DE BREVET EUROPEEN

(45) Date de publication et mention de la délivrance du brevet:
**10.05.2017 Bulletin 2017/19**

(21) Numéro de dépôt: **09179789.4**

(22) Date de dépôt: **18.12.2009**

(51) Int Cl.:
*A61K 8/19* (2006.01) *A61K 8/25* (2006.01)
*A61K 8/22* (2006.01) *A61K 8/49* (2006.01)
*A61K 8/31* (2006.01) *A61K 8/41* (2006.01)
*A61K 8/43* (2006.01) *A61K 8/44* (2006.01)
*A61Q 5/06* (2006.01) *A61Q 5/10* (2006.01)
*A61Q 5/08* (2006.01)

(54) **Procédé d'éclaircissement ou de coloration directe éclaircissante ou d'oxydation en présence d'une amine organique et d'une base minérale et dispositif approprié**

Verfahren zur Aufhellung, aufhellenden Direktfärbung oder Oxidationsfärbung in Gegenwart von organischen Aminen und Mineralbasen, sowie Kit

Method for lightening, lightening direct dyeing or oxidation dyeing carried out in the presence of an organic amine and a mineral base; suitable kit

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **19.12.2008 FR 0807285**
**19.12.2008 FR 0807286**

(43) Date de publication de la demande:
**23.06.2010 Bulletin 2010/25**

(73) Titulaire: **L'Oréal**
**75008 Paris (FR)**

(72) Inventeurs:
- **Hercouet, Leïla**
**93360, Neuilly Plaisance (FR)**
- **Lagrance, Alain**
**77700, Coupvray (FR)**

(74) Mandataire: **Dossmann, Gérard**
**Casalonga & Partners**
**Bayerstrasse 71-73**
**80335 München (DE)**

(56) Documents cités:
**EP-A- 1 374 842** **EP-A- 1 430 873**
**EP-A- 1 438 951** **EP-A- 1 813 254**
**EP-A- 2 072 034** **WO-A-97/01323**
**WO-A-97/04739** **WO-A-2009/080667**
**WO-A-2009/080670** **DE-A1- 3 814 356**
**DE-A1-102005 059 647** **FR-A- 2 132 214**
**FR-A- 2 925 309**

- **DATABASE WPI Week 200470 Thomson Scientific, London, GB; AN 2004-711531 XP002491445 & JP 2004 262886 A (KANEBO LTD) 24 septembre 2004 (2004-09-24)**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 198 831 B1

## Description

La présente invention a pour objet un procédé d'éclaircissement ou un procédé de coloration des fibres kératiniques, en particulier des fibres kératiniques humaines telles que les cheveux en présence d'un ou plusieurs agents oxydants, qui comprend la mise en oeuvre d'une composition cosmétique anhydre (A) comprenant un ou plusieurs corps gras et un ou plusieurs tensioactifs, d'une composition cosmétique (B) comprenant une ou plusieurs amines organiques et une ou plusieurs bases minérales et d'une composition oxydante (C) comprenant un ou plusieurs agents oxydants, lorsque le procédé selon l'invention est un procédé de coloration des fibres kératiniques, la composition (B) comprend en outre un ou plusieurs colorants.

La présente invention concerne aussi un dispositif à plusieurs compartiments comprenant un premier compartiment renfermant la composition cosmétique anhydre (A) précitée, un deuxième compartiment renfermant la composition cosmétique (B) précitée et un troisième compartiment renfermant une composition oxydante (C) précitée.

La présente invention a trait au domaine de la coloration des fibres kératiniques et plus particulièrement au domaine de la coloration capillaire.

Depuis longtemps, de nombreuses personnes cherchent à modifier la couleur de leurs cheveux et en particulier à masquer leurs cheveux blancs. Pour ce faire, il existe essentiellement deux types de coloration qui ont été développés.

Le premier type de coloration est la coloration dite permanente ou d'oxydation qui met en oeuvre des compositions tinctoriales contenant des précurseurs de colorant d'oxydation, appelés généralement bases d'oxydation. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques. La variété des molécules mises en jeu au niveau des bases d'oxydation et des coupleurs permet l'obtention d'une riche palette de couleurs.

Le deuxième type de coloration est la coloration dite semi-permanente ou coloration directe qui consiste à appliquer sur les fibres kératiniques des colorants directs qui sont des molécules colorées et colorantes, ayant une affinité pour les fibres, à laisser pauser pour permettre aux molécules de pénétrer, par diffusion à l'intérieur de la fibre, puis à les rincer.

Afin de réaliser ces colorations, les colorants directs généralement employés sont choisis parmi les colorants directs nitrés benzéniques, anthraquinoniques, nitropyridiniques, azoïques, xanthéniques, acridiniques, aziniques ou triarylméthaniques.

Ce type de procédé ne nécessite pas l'emploi d'un agent oxydant pour développer la coloration. Cependant, il n'est pas exclu d'en mettre en oeuvre afin d'obtenir un effet d'éclaircissement avec la coloration. On parle alors d'une coloration directe ou semi-permanente en conditions éclaircissantes.

Les procédés de coloration permanente ou encore semi-permanente en conditions éclaircissantes, consistent donc à employer avec la composition tinctoriale, une composition aqueuse comprenant au moins un agent oxydant, en condition de pH alcalin dans la grande majorité des cas. Cet agent oxydant a pour rôle, au moins en partie, de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, on met habituellement en oeuvre des sels peroxygénés, comme des persulfates par exemple, en présence de peroxyde d'hydrogène.

Les procédés d'éclaircissement des fibres kératiniques humaines consistent à employer une composition aqueuse comprenant au moins un agent oxydant, en condition de pH alcalin dans la grande majorité des cas. Cet agent oxydant a pour rôle de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, on met habituellement en oeuvre des sels peroxygénés, comme des persulfates par exemple, en présence de peroxyde d'hydrogène.

L'une des difficultés rencontrée lors de la mise en oeuvre des procédés d'éclaircissement ou de coloration éclaircissante dans l'art antérieur vient du fait que ces procédés sont mis en oeuvre dans des conditions alcalines et que l'agent alcalin le plus couramment utilisé est l'ammoniaque. L'emploi de l'ammoniaque est particulièrement avantageux dans ce type de procédés. En effet, il permet d'ajuster le pH de la composition à un pH alcalin pour permettre l'activation de l'agent oxydant. Mais cet agent alcalin provoque également un gonflement de la fibre kératinique, avec un soulèvement des écailles, ce qui favorise la pénétration de l'oxydant, ainsi que des colorants, essentiellement les colorants d'oxydation, à l'intérieur de la fibre, et donc augmente l'efficacité de la réaction de coloration.

Or cet agent alcalinisant est très volatil, ce qui occasionne des désagréments à l'utilisateur du fait de l'odeur caractéristique forte, plutôt incommodante de l'ammoniac qui se dégage durant le procédé.

De plus, la quantité d'ammoniac dégagée nécessite l'emploi de teneurs plus importantes que nécessaires pour compenser cette perte. Cela n'est pas sans conséquence pour l'utilisateur qui reste non seulement incommodé par l'odeur mais qui peut également être confronté à des risques plus importants d'intolérance, comme par exemple une irritation du cuir chevelu se traduisant notamment par des picotements.

Quant à l'option de purement et simplement remplacer en totalité ou en partie l'ammoniaque par un ou plusieurs autres agents alcalinisants classiques, celle-ci ne conduit pas à des compositions aussi efficaces que celles à base d'ammoniaque, notamment parce que ces agents alcalinisants ne conduisent pas un éclaircissement suffisant des fibres pigmentées en présence de l'agent oxydant.

Ainsi l'un des objectifs de la présente invention est de proposer des procédés d'éclaircissement ou de coloration des fibres kératiniques humaines mis en oeuvre en présence d'un agent oxydant qui n'ont pas les inconvénients des procédés existants, dus à la présence de teneurs importantes en ammoniaque, tout en restant au moins aussi efficaces. Les procédés d'éclaircissement doivent être efficaces sur le plan de la qualité et de l'homogénéité de l'éclaircissement, et les procédés de coloration doivent être efficaces sur le plan de la puissance de la coloration obtenue ainsi que sur celui de la chromaticité, de l'homogénéité de la coloration le long de la fibre.

Ces buts et d'autres sont atteints par la présente invention qui a donc pour objet un procédé d'éclaircissement ou de coloration des fibres kératiniques, en particulier des fibres kératiniques humaines telles que les cheveux, en présence d'un agent oxydant dans lequel on applique sur lesdites fibres :

(a) une composition cosmétique anhydre (A) comprenant un ou plusieurs corps gras et un ou plusieurs tensioactifs ;
(b) une composition cosmétique (B) comprenant une ou plusieurs amines organiques dont le $pK_b$ est inférieur à 12 à 25°C et une ou plusieurs bases minérales, et
(c) une composition (C) comprenant un ou plusieurs agents oxydants ;

lorsque le procédé selon l'invention est un procédé de coloration des fibres kératiniques, la composition (B) comprend en outre un ou plusieurs colorants d'oxydation et/ou un ou plusieurs colorants directs.

Le procédé de coloration selon l'invention conduit à des colorations puissantes et peu sélectives, c'est-à-dire à des colorations qui sont homogènes le long de la fibre.

Par ailleurs, les procédés selon l'invention permettent de conduire à des compositions qui ne dégagent pas une odeur agressive lors de leur application sur les cheveux ou au cours de leur préparation.

La présente invention concerne également un dispositif à plusieurs compartiments comprenant dans un premier compartiment une composition cosmétique anhydre (A) comprenant un ou plusieurs corps gras et un ou plusieurs tensioactifs , dans un deuxième compartiment, une composition cosmétique (B) comprenant une ou plusieurs amines organiques dont le $pK_b$ est inférieur à 12 à 25°C et une ou plusieurs bases minérales ainsi qu'éventuellement un ou plusieurs colorants d'oxydation et/ou un ou plusieurs colorants directs et, dans un troisième compartiment, une composition (C) comprenant un ou plusieurs agents oxydants.

Un autre objet de l'invention porte sur une composition anhydre comportant un ou plusieurs corps gras, un ou plusieurs tensioactifs, une ou plusieurs amines organiques dont le $pK_b$ est inférieur à 12 à 25°C et une ou plusieurs bases minérales autres que l'ammoniaque.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Les fibres kératiniques humaines traitées par le procédé selon l'invention sont de préférence les cheveux.

Comme indiqué auparavant, le procédé de coloration est mis en oeuvre en présence d'une composition cosmétique anhydre (A).

Plus particulièrement, on entend, par composition cosmétique anhydre, au sens de l'invention, une composition cosmétique présentant une teneur en eau égale à zéro ou inférieure à 5 % en poids, de préférence inférieure à 2% en poids et de manière encore plus préférée inférieure à 1% en poids par rapport au poids de ladite composition. Il est à noter qu'il s'agit plus particulièrement d'eau liée, comme l'eau de cristallisation des sels ou des traces d'eau absorbée par les matières premières utilisées dans la réalisation des compositions selon l'invention.

Le procédé d'éclaircissement selon l'invention est mis en oeuvre en présence de compositions ne comprenant pas de colorant direct ou de précurseur de colorant d'oxydation (bases et coupleurs) utilisés habituellement pour la coloration des fibres kératiniques humaines ou bien, s'ils sont présents, leur teneur totale ne dépasse pas 0,005 % en poids par rapport au poids de chacune des compositions. En effet, à une telle teneur, seule la composition serait éventuellement teintée, c'est-à-dire qu'on n'observerait pas d'effet de coloration des fibres kératiniques.

De préférence, le procédé d'éclaircissement est mis en oeuvre sans base d'oxydation, ni coupleur, ni colorant direct.

Ainsi que cela a été mentionné, la composition cosmétique anhydre (A) comprend un ou plusieurs corps gras.

Par corps gras, on entend, un composé organique insoluble dans l'eau à température ambiante ordinaire (25°C) et à la pression atmosphérique (760 mm de Hg) (solubilité inférieure à 5% et de préférence à 1% encore plus préférentiellement à 0,1%). Ils présentent dans leur structure au moins une chaîne hydrocarbonée comportant au moins 6 atomes de carbone ou au moins un enchaînement d'au moins deux groupements siloxanes. En outre, les corps gras sont généralement solubles dans les solvants organiques dans les mêmes conditions de température et de pression, comme par exemple le chloroforme, l'éthanol, le benzène, l'huile vaseline ou le décaméthyl cyclopentasiloxane.

Les corps gras sont notamment choisis parmi les alcanes inférieurs en $C_6$-$C_{16}$, les huiles non siliconées animales, végétales, minérales ou synthétiques, les alcools gras, les acides gras, les esters d'acide gras et/ou d'alcool gras, les cires non siliconées et les silicones.

Il est rappelé qu'au sens de l'invention, les alcools, esters et acides gras présentent plus particulièrement un ou plusieurs groupements hydrocarbonés, linéaires ou ramifiés, saturés ou insaturés, comprenant 6 à 30 atomes de carbone, éventuellement substitués, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs en $C_6$-$C_{16}$, ces derniers sont linéaires ou ramifiés, éventuellement cycliques. A titre d'exemple, les alcanes peuvent être choisis parmi l'hexane, l'undécane, le dodécane, le tridécane, les isoparaffines comme l'isohexadécane et l'isodécane.

Comme huiles non siliconées utilisables dans la composition de l'invention, on peut citer par exemple :

- les huiles hydrocarbonées d'origine animale, telles que le perhydrosqualène ;
- les triglycérides d'origine végétale ou synthétique, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;
- les hydrocarbures linéaires ou ramifiés, d'origine minérale ou synthétique, de plus de 16 atomes de carbone, tels que les huiles de paraffine, volatiles ou non, et leurs dérivés, la vaseline, l'huile de vaseline, les polydécènes, les polysiobutènes hydrogénés tels que Parléam ®, de préférence les huiles de paraffine, la vaseline, l'huile de vaseline, les polydécènes, les polyisobutènes hydrogénés tels que Parléam ® ;
- les huiles fluorées partiellement hydrocarbonées ; comme huiles fluorées, on peut citer aussi le perfluorométhylcyclopentane et le perfluoro-1,3 diméthylcyclohexane, vendus sous les dénominations de "FLUTEC® PC1" et "FLUTEC® PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052®" par la Société 3M.

Les alcools gras utilisables dans la composition cosmétique anhydre (A) sont saturés ou insaturés, linéaires ou ramifiés, et comportent de 6 à 30 atomes de carbone et plus particulièrement de 8 à 30 atomes de carbone. On peut citer par exemple l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

La cire ou les cires susceptibles d'être utilisées dans la composition cosmétique anhydre (A) sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les acides gras susceptibles d'être utilisées dans la composition cosmétique anhydre (A) peuvent être saturés ou insaturés et comportent de 6 à 30 atomes de carbone, en particulier de 9 à 30 atomes de carbone. Ils sont choisis plus particulièrement parmi l'acide myristique, l'acide palmitique, l'acide stéarique, l'acide béhénique, l'acide oléique, l'acide linoléique, l'acide linolénique et l'acide isostéarique.

En ce qui concerne les esters d'acide gras et/ou d'alcools gras, avantageusement différents des triglycérides mentionnés ci-dessus, on peut citer notamment les esters de mono ou polyacides aliphatiques saturés ou insaturés, linéaires ou ramifiés en $C_1$-$C_{26}$ et de mono ou polyalcools aliphatiques saturés ou insaturés, linéaires ou ramifiés en $C_1$-$C_{26}$, le nombre total de carbone des esters étant plus particulièrement supérieur ou égal à 10.

Parmi les monoesters, on peut citer le béhénate de dihydroabiétyle ; le béhénate d'octyldodécyle ; le béhénate

d'isocétyle ; le lactate de cétyle ; le lactate d'alkyle en $C_{12}$-$C_{15}$ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en $C_4$-$C_{22}$ et d'alcools en $C_1$-$C_{22}$ et les esters d'acides mono di ou tricarboxyliques et d'alcools di, tri, tétra ou pentahydroxy en $C_2$-$C_{26}$.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en $C_6$-$C_{30}$, de préférence en $C_{12}$-$C_{22}$. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en $C_6$-$C_{30}$, de préférence en $C_{12}$-$C_{22}$, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :

- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le monolaurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

Les silicones utilisables dans la composition cosmétique anhydre (A) de la présente invention, sont des silicones

volatiles ou non volatiles, cycliques, linéaires ou ramifiées, modifiées ou non par des groupements organiques, ayant une viscosité de $5.10^{-6}$ à $2,5 m^2/s$ à 25°C et de préférence $1.10^{-5}$ à $1 m^2/s$.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:

(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.

On peut également citer les cyclocopolymères du type diméthylsiloxanes/ méthylalkylsiloxane, tel que la SILICONE VOLATILE® FZ 3109 commercialisée par la société UNION CARBIDE, de formule :

avec D'' : 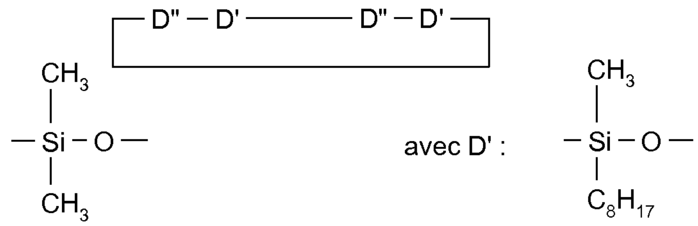 avec D' :

On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;

(ii) les polydialkylsiloxanes volatiles linéaires ayant 2 à 9 atomes de silicium et présentant une viscosité inférieure ou égale à $5.10^{-6} m^2/s$ à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :

- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 $mm^2/s$ ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl ($C_1$-$C_{20}$) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre

200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :

- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6} m^2/s$. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :

$$R_2SiO_{2/2},\ R_3SiO_{1/2},\ RSiO_{3/2} \text{ et } SiO_{4/2}$$

dans lesquelles R représente un alkyl possédant 1 à 16 atomes de carbone. Parmi ces produits, ceux particulièrement préférés sont ceux dans lesquels R désigne un groupe alkyle inférieur en $C_1$-$C_4$, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de $1.10^{-5}$ à $5.10^{-2} m^2/s$ à 25°C.

Parmi ces polyalkylarylsiloxanes, on peut citer à titre d'exemple les produits commercialisés sous les dénominations suivantes :

- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl ($C_{12}$)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence, les corps gras ne comprennent pas de motif oxyalkyléné en $C_2$-$C_3$ ni de motif glycérolé.
Plus particulièrement, les corps gras sont choisis parmi les composés liquides ou pâteux à température ambiante et à pression atmosphérique.
Plus particulièrement, les corps gras sont différents des acides gras.
Les corps gras sont de préférence choisis parmi les alcanes inférieurs en $C_6$-$C_{16}$, les huiles non siliconées minérales, végétales ou synthétiques, les alcools gras, les esters d'acide gras et/ou d'alcool gras, les silicones.
De préférence, le ou les corps gras de la composition selon l'invention sont non siliconés.
De préférence, le ou les corps gras est ou sont choisis parmi l'huile de vaseline, les polydécènes, les esters d'acides gras et/ou d'alcools gras, liquides ou leurs mélanges.
La composition cosmétique anhydre (A) présente une teneur en corps gras comprise entre 20 et 90 % en poids, et plus particulièrement entre 25 et 80% en poids, par rapport au poids de la composition anhydre (A).
La composition anhydre cosmétique (A) comprend également un ou plusieurs tensioactifs.
Plus particulièrement, le ou les tensioactifs sont choisis parmi les tensioactifs non ioniques ou parmi les tensioactifs anioniques.
Les tensioactifs anioniques sont plus spécialement choisis parmi les sels (en particulier sels de métaux alcalins, notamment de sodium, sels d'ammonium ou sels de métaux alcalino-terreux comme le magnésium) des composés suivants :

- les alkylsulfates, les alkyléthersulfates, alkylamidoéthersulfates, alkylaryl-polyéthersulfates, monoglycérides sulfates ;
- les alkylsulfonates, alkylamidesulfonates, alkylarylsulfonates, $\alpha$-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.
Les tensioactifs non ioniques sont plus particulièrement choisis parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.
A titre d'exemples de tensioactifs non ioniques oxyalkylénés, on peut citer :

- les alkyl($C_8$-$C_{24}$)phénols oxyalkylénés,
- les alcools en $C_8$-$C_{30}$, saturés ou non, linéaires ou ramifiés, oxyalkylénés,

- les amides, en $C_8$-$C_{30}$, saturés ou non, linéaires ou ramifiés, oxyalkylénés,
- les esters d'acides en $C_8$-$C_{30}$, saturés ou non, linéaires ou ramifiés, et de polyéthylèneglycols,
- les esters d'acides en $C_8$-$C_{30}$, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres,
- ou leurs mélanges.

De préférence, les tensioactifs présentent un nombre de moles d'oxyde d'éthylène et/ou de propylène compris entre 1 et 100, de préférence entre 2 et 50. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en $C_8$-$C_{30}$, oxyéthylénés, les esters d'acides en $C_8$-$C_{30}$, saturés ou non, linéaires ou ramifiés, et de sorbitol polyoxyéthylénés.

A titre d'exemple de tensioactifs non ioniques mono- ou poly-glycérolés, on utilise de préférence les alcools en $C_8$-$C_{40}$, mono- ou poly- glycérolés.

En particulier, les alcools en $C_8$-$C_{40}$ mono- ou poly- glycérolés correspondent à la formule suivante :

$$RO\text{-}[CH_2\text{-}CH(CH_2OH)\text{-}O]_m\text{-}H$$

dans laquelle R représente un radical alkyle ou alcényle, linéaire ou ramifié, en $C_8$-$C_{40}$, de préférence en $C_8$-$C_{30}$, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en $C_8/C_{10}$ à une mole de glycérol, l'alcool en $C_{10}/C_{12}$ à 1 mole de glycérol et l'alcool en $C_{12}$ à 1,5 mole de glycérol.

De préférence, le tensioactif présent dans la composition anhydre est un tensioactif non ionique.

La teneur en tensioactifs dans la composition anhydre (A), représente plus particulièrement de 0,1 à 50 % en poids, de préférence de 0,5 à 30 % en poids par rapport au poids de la composition anhydre.

La composition anhydre (A) peut également renfermer divers adjuvants utilisés classiquement dans les compositions pour la coloration des cheveux, tels que des polymères anioniques, cationiques, non ioniques, amphotères, zwitterioniques ou leurs mélanges ; des agents épaississants minéraux, et en particulier des charges telles que des argiles, le talc ; des agents épaississants organiques, avec en particulier les épaississants associatifs polymères anioniques, cationiques, non ioniques et amphotères différents des polymères cités précédemment ; des agents antioxydants ; des agents de pénétration ; des agents séquestrants ; des parfums ; des agents dispersants ; des agents filmogènes ; des céramides ; des agents conservateurs ; des agents opacifiants.

Les adjuvants ci dessus sont en général présents en quantité comprise pour chacun d'eux entre 0,01 et 20 % en poids par rapport au poids de la composition (A).

Selon une variante avantageuse de l'invention, la composition anhydre (A) comprend un ou plusieurs agents épaississants minéraux choisis parmi les argiles organophiles, les silices pyrogénées, ou leurs mélanges.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles

vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay, les Quaternium-18 Hectorites telles que celles vendues sous les dénominations Bentone Gel DOA, Bentone Gel ECO5, Bentone Gel EUG, Bentone Gel IPP, Bentone Gel ISD, Bentone Gel SS71, Bentone Gel VS8, Bentone Gel VS38 par la société Rhéox et Simagel M, Simagel SI 345 par la société Biophil.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de la silice par réaction chimique en vue de diminuer le nombre de groupes silanol. On peut notamment substituer des groupes silanol par des groupements hydrophobes : on obtient alors une silice hydrophobe. Les groupements hydrophobes peuvent être :

- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

De préférence, la composition comprend une hectorite, une bentonite organomodifiée ou une silice pyrogénée éventuellement modifiée.

Lorsqu'il est présent, l'agent épaississant minéral représente de 1 à 30 % en poids par rapport au poids de la composition.

Avantageusement, la composition se présente sous la forme d'un gel ou d'une crème.

Comme indiqué précédemment, dans le cas de la mise en oeuvre d'un procédé d'éclaircissement, la composition (B) ne comprend pas de colorant direct ou de précurseur de colorant d'oxydation (bases et coupleurs) ou bien, s'ils sont présents, leur teneur totale ne dépasse pas 0,005% en poids par rapport au poids de la composition (B), de préférence le procédé d'éclaircissement est mis en oeuvre sans base d'oxydation, ni coupleur, ni colorant direct.

Le procédé de coloration selon l'invention est mis en oeuvre en présence d'une composition cosmétique (B) comprenant un ou plusieurs colorants d'oxydation, un ou plusieurs colorants directs, ou leurs mélanges.

Les colorants d'oxydation sont en général choisis parmi les bases d'oxydation éventuellement combinée(s) à un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diaminol-(β-méthoxyéthyl)pyrazole.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydropyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels.

La composition cosmétique (B) selon l'invention peut éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(ß-hydroxyéthyloxy) benzène, le 2-amino 4-(ß-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-ß-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(ß-hydroxyéthyl)amino-3,4-méthylène dioxybenzène, le 2,6-bis-(ß-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent chacune avantageusement de 0,0001 à 10 % en poids par rapport au poids total de la composition, et de préférence de 0,005 à 5 % en poids par rapport au poids total de la composition.

La teneur en coupleur(s), s'il(s) est (sont) présent(s), représentent chacun avantageusement de 0,0001 à 10 % en poids par rapport au poids total de la composition, et de préférence de 0,005 à 5 % en poids par rapport au poids total de la composition cosmétique (B).

En ce qui concerne les colorants directs, ces derniers sont plus particulièrement choisis parmi les espèces ioniques ou non ioniques, de préférence cationiques ou non ioniques.

A titre d'exemples de colorants directs convenables, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Plus particulièrement, les colorants azoïques comprennent une fonction -N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine, azométhine, mono- et di-arylméthane, indoamines (ou diphénylamines), indophénols, indoanilines, carbocyanines, azacabocyanines et leurs isomères, diazacarbocyanines et leurs isomères, tétraazacarbocyanines, hémicyanines

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des azines cycliques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs particulièrement convenables, on peut citer les colorants nitrés de la série benzénique ; les colorants directs azoïques ; azométhiniques ; méthiniques ; les azacarbocyanines comme les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines, porphyrines et les colorants directs naturels, seuls ou en mélanges.

Ces colorants peuvent être des colorants monochromophoriques (c'est-à-dire ne comprenant qu'un seul colorant) ou polychromophoriques, de préférence di- ou tri- chromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

De préférence, le bras de liaison est une chaîne alkyle en $C_1$-$C_{20}$, linéaire, ramifiée ou cyclique, éventuellement interrompue par au moins un hétéroatome (tel que l'azote, l'oxygène) et/ou par au moins un groupe en comprenant (CO, $SO_2$), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en $C_1$-$C_{15}$ éventuellement substitués ; le bras de liaison ne comprenant pas de groupement nitro, nitroso ou peroxo.

Si les hétérocycles ou noyaux aromatiques sont substitués, ils le sont par exemple par un ou plusieurs radicaux alkyle en $C_1$-$C_8$ éventuellement substitués par un groupement hydroxy, alcoxy en $C_1$-$C_2$, hydroxyalcoxy en $C_2$-$C_4$, acétylamino, amino substitué par un ou deux radicaux alkyle en $C_1$-$C_4$, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle ; un radical alcoxy en $C_1$-$C_2$ ; un radical hydroxyalcoxy en $C_2$-$C_4$ ; un radical amino ; un radical amino substitué par un ou deux radicaux alkyle, identiques ou différents, en $C_1$-$C_4$ éventuellement porteurs d'au moins un groupement hydroxyle.

Parmi les colorants directs benzéniques utilisables selon l'invention, on peut citer de manière non limitative les composés suivants :

- 1,4-diamino-2-nitrobenzène,
- 1-amino-2 nitro-4-ß- hydroxyéthylaminobenzène
- 1-amino-2 nitro-4-bis(β-hydroxyéthyl)-aminobenzène
- 1,4-bis(ß -hydroxyéthylamino)-2-nitrobenzène
- 1-ß-hydroxyéthylamino-2-nitro-4-bis-(β-hydroxyéthylamino)-benzène
- 1-ß-hydroxyéthylamino-2-nitro-4-aminobenzène
- 1-β-hydroxyéthylamino-2-nitro-4-(éthyl)(ß-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines, méthines ou tétraazapentaméthines utilisables selon l'invention on peut citer les colorants cationiques décrits dans les demandes de brevets WO 95/15144, WO 95/01772 et EP 714954 ; FR 2189006, FR 2285851, FR-2140205, EP 1378544, EP 1674073.

Ainsi, on peut tout notamment citer les colorants suivants de formules (I) à (IV), et de préférence les composés de formules (I) et (III) :

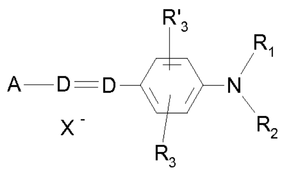

(I)

dans laquelle :

D représente un atome d'azote ou le groupement -CH,

$R_1$ et $R_2$, identiques ou différents, représentent un atome d'hydrogène ; un radical alkyle en $C_1$-$C_4$ pouvant être substitué par un radical -CN, -OH ou -$NH_2$ ou forment avec un atome de carbone du cycle benzénique un hétérocycle éventuellement oxygéné ou azoté, pouvant être substitué par un ou plusieurs radicaux alkyle en $C_1$-$C_4$ ; un radical 4'-aminophényle,

$R_3$ et $R'_3$, identiques ou différents, représentent un atome d'hydrogène ou d'halogène choisi parmi le chlore, le brome, l'iode et le fluor, un radical cyano, alkyl en $C_1$-$C_4$ , alcoxy en $C_1$-$C_4$ ou acétyloxy,

$X^-$ représente un anion de préférence choisi parmi le chlorure, le méthyl sulfate et l'acétate,

A représente un groupement choisi par les structures A1 à A18, de préférence A1, A4, A7, A13 et A18, suivantes :

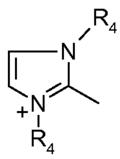 ; 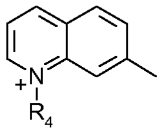 ; 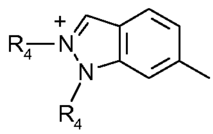 ;

$A_1$ $A_2$ $A_3$

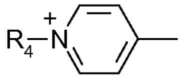 ; 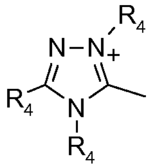 ; 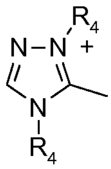 ;

$A_4$ $A_5$ $A_6$

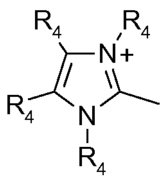 ; 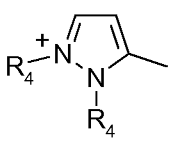 ; 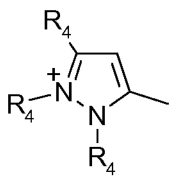 ;

$A_7$ $A_8$ $A_9$

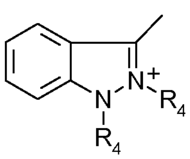 ; 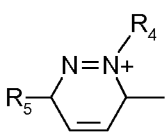 ; 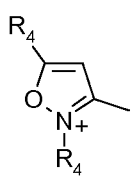 ;

$A_{10}$ 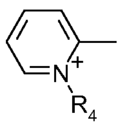 ; $A_{11}$ 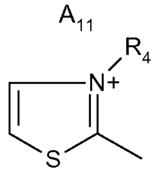 ; $A_{12}$ 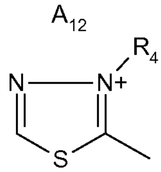 ;

$A_{13}$ $A_{14}$ $A_{15}$

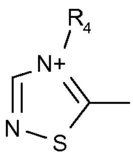 ; 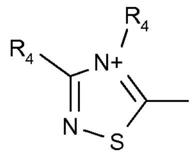 ; 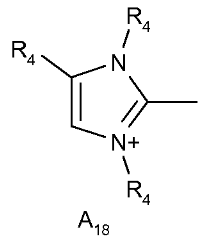

$A_{16}$ $A_{17}$ $A_{18}$

dans lesquelles $R_4$ représente un radical alkyle en $C_1$-$C_4$ pouvant être substitué par un radical hydroxyle et $R_5$ représente un radical alcoxy en $C_1$-$C_4$ ;

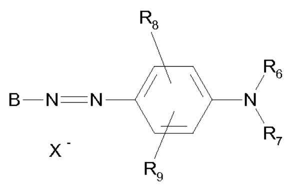 (II)

dans laquelle :

$R_6$ représente un atome d'hydrogène ou un radical alkyle en $C_1$-$C_4$,
$R_7$ représente un atome d'hydrogène, un radical alkyle pouvant être substitué par un radical -CN ou par un groupement amino, un radical 4'-aminophényle ou forme avec $R_6$ un hétérocycle éventuellement oxygéné et/ou azoté pouvant être substitué par un radical alkyle en $C_1$-$C_4$,
$R_8$ et $R_9$, identiques ou différents, représentent un atome d'hydrogène, un atome d'halogène tel que le brome, le chlore, l'iode ou le fluor, un radical alkyle en $C_1$-$C_4$ ou alcoxy en $C_1$-$C_4$, un radical - CN,
$X^-$ représente un anion de préférence choisi parmi le chlorure, le méthyl sulfate et l'acétate,
B représente un groupement choisi par les structures B1 à B6 suivantes :

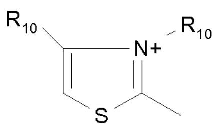 ; 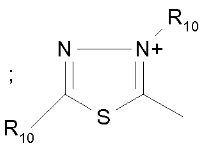 ; 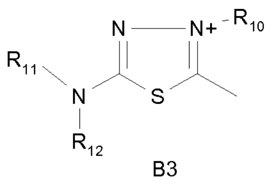 ;

B1 B2 B3

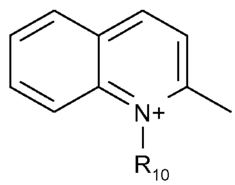 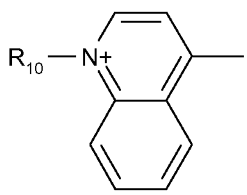 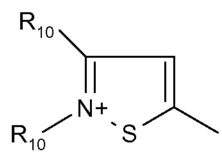

B4 B5 B6

dans lesquelles $R_{10}$ représente un radical alkyle en $C_1$-$C_4$, $R_{11}$ et $R_{12}$, identiques ou différents, représentent un

atome d'hydrogène ou un radical alkyle en $C_1$-$C_4$ ;

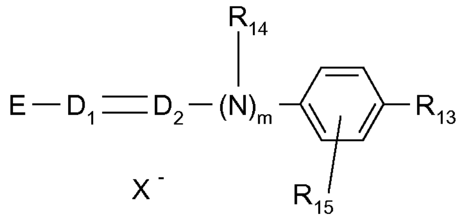

(III)

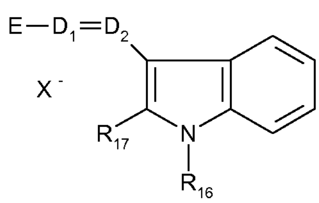

(III')

dans lesquelles :

$R_{13}$ représente un atome d'hydrogène, un radical alcoxy en $C_1$-$C_4$, un atome d'halogène tel que le brome, le chlore, l'iode ou le fluor,

$R_{14}$ représente un atome d'hydrogène, un radical alkyle en $C_1$-$C_4$ ou forme avec un atome de carbone du cycle benzénique un hétérocycle éventuellement oxygéné et/ou substitué par un ou plusieurs groupements alkyle en $C_1$-$C_4$,

$R_{15}$ représente un atome d'hydrogène ou d'halogène tel que le brome, le chlore, l'iode ou le fluor,

$R_{16}$ et $R_{17}$, identiques ou différents, représentent un atome d'hydrogène ou un radical alkyle en $C_1$-$C_4$,

$D_1$ et $D_2$, identiques ou différents, représentent un atome d'azote ou le groupement -CH,

m = 0 ou 1, de préférence 1,

étant entendu que lorsque $R_{13}$ représente un groupement amino non substitué, alors $D_1$ et $D_2$ représentent simultanément un groupement -CH et m = 0,

$X^-$ représente un anion de préférence choisi parmi le chlorure, le méthyl sulfate et l'acétate,

E représente un groupement choisi par les structures E1 à E8, plus particulièrement E1, E2 et E7, suivantes :

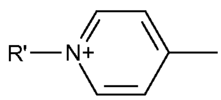

E1

;

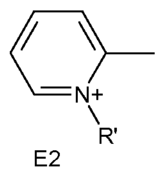

E2

;

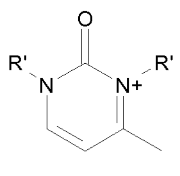

E3

;

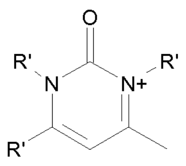

E4

;

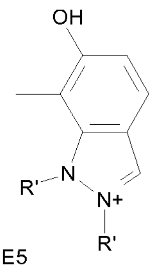

E5

;

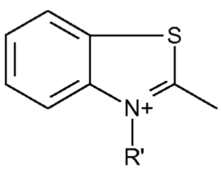

E6

;

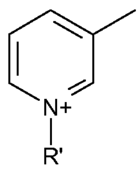

E7

et

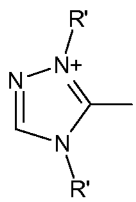

E8

;

dans lesquelles R' représente un radical alkyle en $C_1$-$C_4$ ;

lorsque m = 0 et que $D_1$ représente un atome d'azote, alors E peut également désigner un groupement de structure E9 suivante :

E9 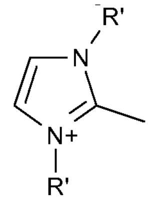 ;

dans laquelle R' représente un radical alkyle en $C_1$-$C_4$.

G-N=N-J (IV)

dans laquelle :

le symbole G représente un groupement choisi parmi les structures $G_1$ à $G_3$ suivantes :

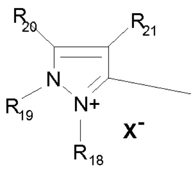

$G_1$

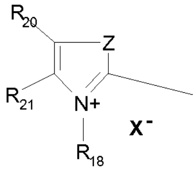

$G_2$

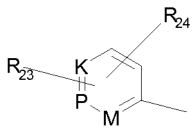

$G_3$

structures $G_1$ à $G_3$ dans lesquelles,

$R_{18}$ désigne un radical alkyle en $C_1$-$C_4$, un radical phényle pouvant être substitué par un radical alkyle en $C_1$-$C_4$ ou un atome d'halogène choisi parmi le chlore, le brome, l'iode et le fluor ;
$R_{19}$ désigne un radical alkyle en $C_1$-$C_4$ ou un radical phényle;
$R_{20}$ et $R_{21}$, identiques ou différents, représentent un radical alkyle en $C_1$-$C_4$, un radical phényle, ou forment ensemble dans $G_1$ un cycle benzénique substitué par un ou plusieurs radicaux alkyle en $C_1$-$C_4$ , alcoxy en $C_1$-$C_4$, ou $NO_2$, ou forment ensemble dans $G_2$ un cycle benzénique éventuellement substitué par un ou plusieurs radicaux alkyle en $C_1$-$C_4$ , alcoxy en $C_1$-$C_4$, ou $NO_2$;
$R_{20}$ peut désigner en outre un atome d'hydrogène;
Z désigne un atome d'oxygène, de soufre ou un groupement - $NR_{19}$;
M représente un groupement -CH, -CR (R désignant alkyle en $C_1$-$C_4$),
ou -$NR_{22}(X^-)_r$;
K représente un groupement -CH, -CR (R désignant alkyle en $C_1$-$C_4$),
ou -$NR_{22}(X^-)_r$;
P représente un groupement -CH, -CR (R désignant alkyle en $C_1$-$C_4$),
ou -$NR_{22}(X^-)_r$; r désigne zéro ou 1;
$R_{22}$ représente un atome $O^-$, un radical alcoxy en $C_1$-$C_4$, ou un radical alkyle en $C_1$-$C_4$;
$R_{23}$ et $R_{24}$, identiques ou différents, représentent un atome d'hydrogène ou d'halogène choisi parmi le chlore, le brome, l'iode et le fluor, un radical alkyle en $C_1$-$C_4$, alcoxy en $C_1$-$C_4$ , un radical - $NO_2$;
$X^-$ représente un anion de préférence choisi parmi le chlorure, l'iodure, le méthyl sulfate, l'éthyl sulfate, l'acétate et le perchlorate;
sous réserve que,

si $R_{22}$ désigne $O^-$, alors r désigne zéro;
si K ou P ou M désignent -N-alkyle $C_1$-$C_4$ $X^-$ , alors $R_{23}$ ou $R_{24}$ est de préférence différent d'un atome d'hydrogène ;
si K désigne -$NR_{22}(X^-)_r$, alors M= P= -CH, -CR;
si M désigne -$NR_{22}(X^-)_r$, alors K= P= -CH, -CR;
si P désigne -$NR_{22}(X^-)_r$, alors K= M et désignent -CH ou -CR;
si Z désigne un atome de soufre avec $R_{21}$ désignant alkyle en $C_1$-$C_4$ , alors $R_{20}$ est différent d'un atome

d'hydrogène;
si Z désigne $-NR_{22}$ avec $R_{19}$ désignant alkyle en $C_1$-$C_4$, alors au moins l'un des radicaux $R_{18}$, $R_{20}$ ou $R_{21}$ du groupement de structure $G_2$ est différent d'un radical alkyle en $C_1$-$C_4$;

le symbole J représente :

- (a) un groupement de structure $J_1$ suivante :

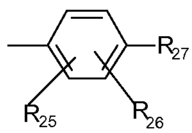

**$J_1$**

structure $J_1$ dans laquelle,

$R_{25}$ représente un atome d'hydrogène, un atome d'halogène choisi parmi le chlore, le brome, l'iode et le fluor, un radical alkyle en $C_1$-$C_4$, alcoxy en $C_1$-$C_4$, un radical -OH, $-NO_2$, $-NHR_{28}$, $-NR_{29}R_{30}$, - NHCOalkyle en $C_1$-$C_4$, ou forme avec $R_{26}$ un cycle à 5 ou 6 chaînons contenant ou non un ou plusieurs hétéroatomes choisis parmi l'azote, l'oxygène ou le soufre;
$R_{26}$ représente un atome d'hydrogène, un atome d'halogène choisi parmi le chlore, le brome, l'iode et le fluor, un radical alkyle en $C_1$-$C_4$, alcoxy en $C_1$-$C_4$,
ou forme avec $R_{27}$ ou $R_{28}$ un cycle à 5 ou 6 chaînons contenant ou non un ou plusieurs hétéroatomes choisis parmi l'azote, l'oxygène ou le soufre;
$R_{27}$ représente un atome d'hydrogène, un radical -OH, un radical $-NHR_{28}$, un radical $-NR_{29}R_{30}$;
$R_{28}$ représente un atome d'hydrogène, un radical alkyle en $C_1$-$C_4$, un radical monohydroxyalkyle en $C_1$-$C_4$, polyhydroxyalkyle en $C_2$-$C_4$, un radical phényle;
$R_{29}$ et $R_{30}$, identiques ou différents, représentent un radical alkyle en $C_1$-$C_4$, un radical monohydroxyalkyle en $C_1$-$C_4$, polyhydroxyalkyle en $C_2$-$C_4$;

- (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en $C_1$-$C_4$, amino ou phényle, et notamment un groupement de structure $J_2$ suivante :

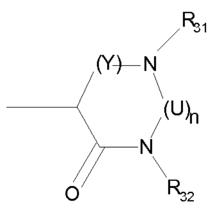

**$J_2$**

structure $J_2$ dans laquelle,

$R_{31}$ et $R_{32}$, identiques ou différents, représentent un atome d'hydrogène, un radical alkyle en $C_1$-$C_4$, un radical phényle;

Y désigne le radical -CO- ou le radical

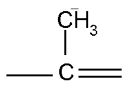 ;

n = 0 ou 1, avec, lorsque n désigne 1, U désigne le radical - CO-.

Dans les structures (I) à (IV) définies ci-dessus le groupement alkyle ou alcoxy en $C_1$-$C_4$ désigne de préférence méthyle, éthyle, butyle, méthoxy, éthoxy.

Parmi les composés de formules (I) et (III), on préfère les composés suivants :

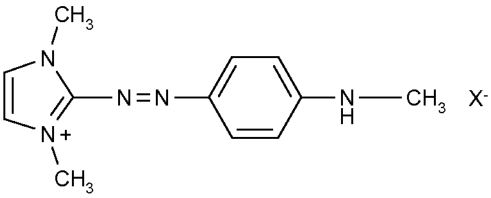

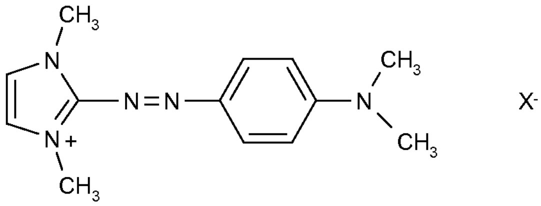

,

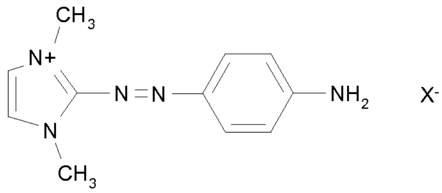

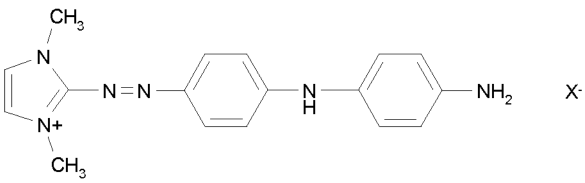

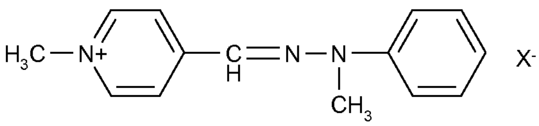

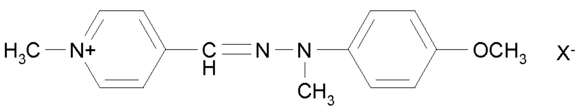

;

On peut également citer parmi les colorants directs azoïques les colorants suivants, décrits dans le COLOUR INDEX INTERNATIONAL 3e édition :

- Disperse Red 17
- Basic Red 22
- Basic Red 76
- Basic Yellow 57
- Basic Brown 16
- Basic Brown 17
- Disperse Black 9.

On peut aussi citer le 1-(4'-aminodiphénylazo)-2-méthyl-4bis-(β-hydroxyéthyl) aminobenzène.

Parmi les colorants directs quinoniques on peut citer les colorants suivants :

- Disperse Red 15
- Solvent Violet 13
- Disperse Violet 1

- Disperse Violet 4
- Disperse Blue 1
- Disperse Violet 8
- Disperse Blue 3
- Disperse Red 11
- Disperse Blue 7
- Basic Blue 22
- Disperse Violet 15
- Basic Blue 99

ainsi que les composés suivants :

- 1-N-méthylmorpholiniumpropylamino-4-hydroxy anthraquinone
- 1-Aminopropylamino-4-méthylaminoanthraquinone
- 1-Aminopropylamino anthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :

- Basic Blue 17
- Basic Red 2.

Parmi les colorants triarylméthaniques utilisables selon l'invention, on peut citer les composés suivants :

- Basic Green 1
- Basic Violet 3
- Basic Violet 14
- Basic Blue 7
- Basic Blue 26

Parmi les colorants indoaminiques utilisables selon l'invention, on peut citer les composés suivants :

- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous, An étant défini comme précédemment :

| | |
|---|---|
| 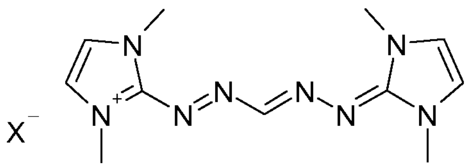 | 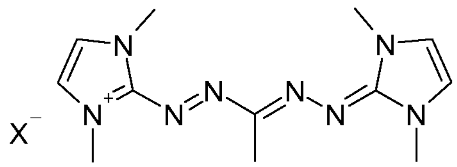 |
| 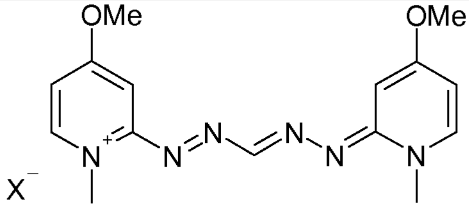 | 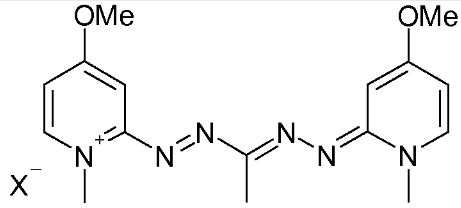 |
| 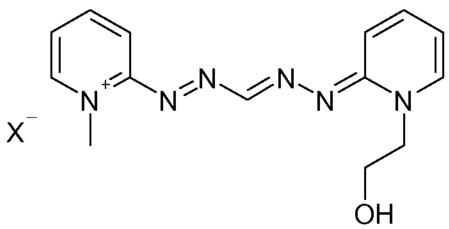 | 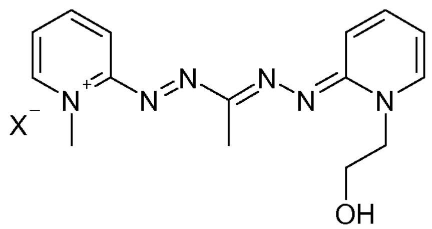 |
| 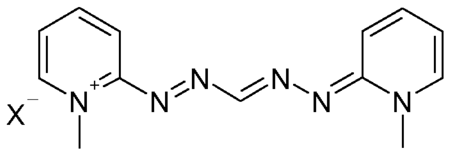 | 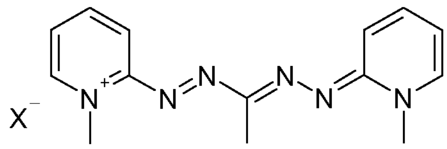 |
| 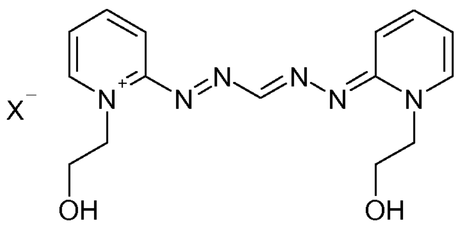 | 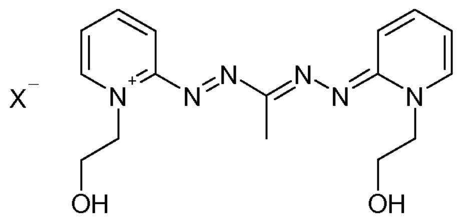 |

X représente un anion de préférence choisi parmi le chlorure, l'iodure, le méthyl sulfate, l'éthyl sulfate, l'acétate et le perchlorate.

Parmi les colorants polychromophoriques, on peut citer plus particulièrement les colorants di- ou tri- chromophoriques azoïques et/ou azométhiniques (hydrazoniques), symétriques ou non, comprenant d'une part au moins un hétérocycle aromatique comprenant 5 ou 6 chaînons, éventuellement condensé, comprenant au moins un atome d'azote quaternisé engagé dans ledit hétérocycle et éventuellement au moins un autre hétéroatome (tel que l'azote, le soufre, l'oxygène), et d'autre part, au moins un groupement phényle ou naphtyle, éventuellement substitué, éventuellement porteur d'au moins un groupement OR avec R représentant un atome d'hydrogène, un radical alkyle éventuellement substitué en $C_1$-$C_6$, un noyau phényle éventuellement substitué, ou d'au moins un groupement $N(R')_2$ avec R' identiques ou non, représentant un atome d'hydrogène, un radical alkyle éventuellement substitué en $C_1$-$C_6$, un noyau phényle éventuellement substitué ; les radicaux R' pouvant former avec l'atome d'azote auquel ils sont liés, un hétérocycle saturé à 5 ou 6 chaînons, ou bien encore l'un et/ou les deux radicaux R' peuvent former chacun avec l'atome de carbone du cycle aromatique placé en ortho de l'atome d'azote, un hétérocycle saturé à 5 ou 6 chaînons.

A titre d'hétérocycle cationique aromatique, on peut citer de préférence, les cycles à 5 ou 6 chaînons comprenant 1 à 3 atomes d'azote, de préférence 1 ou 2 atomes d'azote, l'un étant quaternisé ; ledit hétérocycle étant par ailleurs éventuellement condensé à un noyau benzénique. Il est à noter de même que l'hétérocycle peut éventuellement comprendre un autre hétéroatome différent de l'azote, comme le soufre ou l'oxygène.

Si les hétérocycles ou groupements phényle ou napthyle sont substitués, ils le sont par exemple par un ou plusieurs radicaux alkyle en $C_1$-$C_8$ éventuellement substitués par un groupement hydroxy, alcoxy en $C_1$-$C_2$, hydroxyalcoxy en $C_2$-$C_4$, acétylamino, amino substitué par un ou deux radicaux alkyle en $C_1$-$C_4$, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle ; un radical alcoxy en $C_1$-$C_2$ ; un radical hydroxyalcoxy en $C_2$-$C_4$ ; un radical amino ; un radical amino substitué par un ou deux radicaux alkyle, identiques ou différents, en $C_1$-$C_4$ éventuellement porteurs d'au moins un groupement hydroxyle.

Ces polychromophores sont reliés entre eux au moyen d'au moins un bras de liaison comprenant éventuellement au moins un atome d'azote quaternisé engagé ou non dans un hétérocycle saturé ou non, éventuellement aromatique.

De préférence, le bras de liaison est une chaîne alkyle en $C_1$-$C_{20}$, linéaire, ramifiée ou cyclique, éventuellement interrompue par au moins un hétéroatome (tel que l'azote, l'oxygène) et/ou par au moins un groupe en comprenant (CO, $SO_2$), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en $C_1$-$C_{15}$ éventuellement substitués ; le bras de liaison ne comprenant pas de groupement nitro, nitroso ou peroxo.

La liaison entre le bras de liaison et chaque chromophore se fait en général au moyen d'un hétéroatome substituant le noyau phényle ou napthyle ou au moyen de l'atome d'azote quaternisé de l'hétérocycle cationique.

Le colorant peut comprendre des chromophores identiques ou non.

A titre d'exemples de tels colorants, on pourra notamment se reporter aux demandes de brevets EP 1637566, EP 1619221, EP 1634926, EP 1619220, EP 1672033, EP 1671954, EP 1671955, EP 1679312, EP 1671951, EP167952, EP167971, WO 06/063866, WO 06/063867, WO 06/063868, WO 06/063869, EP 1408919, EP 1377264, EP 1377262, EP 1377261, EP 1377263, EP 1399425, EP 1399117, EP 1416909, EP 1399116, EP 1671560.

On peut aussi également mettre en oeuvre des colorants directs cationiques cités dans les demandes EP 1006153, qui décrit des colorants comprenant deux chromophores de type anthraquinones reliés au moyen d'un bras de liaison cationique ; EP 1433472, EP 1433474, EP 1433471 et EP 1433473 qui décrivent des colorants dichromophoriques identiques ou non, reliés par un bras de liaison cationique ou non, ainsi que EP 6291333 qui décrit notamment des colorants comprenant trois chromophores, l'un d'eux étant un chromophore anthraquinone auquel sont reliés deux chromophores de type azoïque ou diazacarbocyanine ou l'un de ses isomères.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Lorsqu'ils sont présents, le ou les colorants directs représentent plus particulièrement de 0,0001 à 10 % en poids du poids total de la composition, et de préférence de 0,005 à 5 % en poids.

La composition cosmétique (B) peut comprendre l'un et ou l'autre types de colorants. Elle peut éventuellement être issue du mélange de deux compositions colorantes l'une comprenant le ou les colorants d'oxydation, l'autre, le ou les colorants directs.

La composition cosmétique (B) comprend en outre une ou plusieurs amines organiques dont le $pK_b$ est inférieur à 12 à 25°C.

Le ou les amines organiques présentent un $pK_b$ à 25°C qui est inférieur à 12, de préférence inférieur à 10, et encore plus préférentiellement inférieur à 6.

Il est à noter qu'il s'agit du $pK_b$ correspondant à la fonction de basicité la plus élevée.

Selon une première variante de l'invention, l'amine organique comprend une fonction amine primaire, secondaire ou tertiaire, et un ou plusieurs groupements alkyle, linéaires ou ramifiés, en $C_1$-$C_8$ porteurs d'un ou plusieurs radicaux hydroxyle.

Conviennent en particulier, les amines organiques choisies parmi les alcanolamines telles que les mono-, di- ou tri-alcanolamines, comprenant un à trois radicaux hydroxyalkyle, identiques ou non, en $C_1$-$C_4$.

Parmi des composés de ce type, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylamino méthane.

Conviennent également les amines organiques de formule suivante :

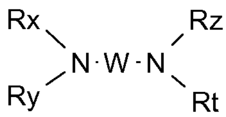

dans laquelle W est un reste alkylène en $C_1$-$C_6$ éventuellement substitué par un groupement hydroxyle ou un radical alkyle en $C_1$-$C_6$, Rx, Ry, Rz et Rt, identiques ou différents, représentent un atome d'hydrogène, un radical alkyle en $C_1$-$C_6$ ou hydroxyalkyle en $C_1$-$C_6$, aminoalkyle en $C_1$-$C_6$.

On peut citer à titre d'exemple de telles amines, le 1,3 diaminopropane, le 1,3 diamino 2 propanol, la spermine, la spermidine.

Selon une deuxième variante de l'invention, l'amine organique est choisie parmi les acides aminés.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (I) suivante :

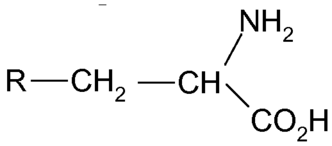

**(I)**

où R désigne un groupe choisi parmi :

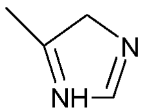

;

$-(CH_2)_3NH_2$ ;
$-(CH_2)_2NH_2$ ; $-(CH_2)_2NHCONH_2$ ;

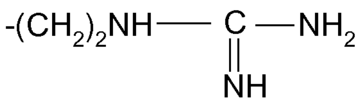

Les composés correspondants à la formule (I) sont l'histidine, la lysine, l'arginine, l'ornithine, la citrulline.

Selon une troisième variante de l'invention, l'amine organique est choisie parmi les amines organiques de type hétérocycliques. On peut en particulier citer, outre l'histidine déjà mentionnée dans les acides aminés, la pyridine, la pipéridine, l' imidazole, le triazole, le tétrazole, le benzimidazole .

Selon une quatrième variante de l'invention, l'amine organique est choisie parmi les dipeptides d'acides aminés. A titre de dipeptides d'acides aminés utilisables dans la présente invention, on peut notamment citer la carnosine, l'anserine et la baleine.

Selon une cinquième variante de l'invention, l'amine organique est choisie parmi les composés comportant une fonction guanidine. Ainsi l'amine organique peut être choisi parmi la guanidine, l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino)méthyl]amino)éthane-1-sulfonique.

De préférence, le ou les amines organiques sont choisies parmi les alcanolamines, les acides aminés basiques, les composés comportant une fonction guanidine.

En particulier, le ou les amines organiques sont des alcanolamines choisies parmi la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylamino méthane

De manière encore plus préférée, le ou les amines organiques sont des alcanolamines, en particulier la monoéthanolamine.

De manière avantageuse, la composition cosmétique (B) présente une teneur en amine(s) organique(s) allant de 0,01 à 30% en poids, de préférence de 0,1 à 20% en poids par rapport au poids de ladite composition.

La composition cosmétique (B) comprend également une ou plusieurs bases minérales.

Par composé minéral, au sens de la présente invention, on entend tout composé possédant dans sa structure un ou plusieurs éléments des colonnes 1 à 13 du tableau périodique des éléments autres que l'hydrogène.

Selon un mode de réalisation particulier de l'invention, la base minérale contient un ou plusieurs éléments des colonnes 1 et 2 du tableau périodique des éléments autre que l'hydrogène.

Dans une variante préférée, la base minérale présente la structure suivante :

$$(Z_1^{x-})_m(Z_2^{y+})_n$$

Dans laquelle :

$Z_2$ désigne un métal des colonnes 1 à 13 du tableau périodique des éléments, de préférence 1 ou 2, comme le sodium ou le potassium ;
$Z_1^{x-}$ désigne un anion choisi parmi les ions $CO_3^{2-}$, $OH^-$, $HCO_3^{2-}$, $SiO_3^{2-}$, $HPO_4^{2-}$, $PO_4^{3-}$, $B_4O_7^{2-}$, de préférence parmi les ions $CO_3^{2-}$, $OH^-$, $SiO_3^{2-}$ ;
x désigne 1 , 2 ou 3 ;
y désigne 1, 2, 3 ou 4 ;
m et n désignent indépendamment l'un de l'autre 1, 2, 3 ou 4 ;

avec n.y=m.x.

De préférence, la base minérale correspond à la formule suivante $(Z_1^{x-})_m(Z_2^{y+})_n$, dans laquelle $Z_2$ désigne un métal des colonnes 1 et 2, du tableau périodique des éléments ; $Z_1^{x-}$ désigne un anion choisi parmi les ions $CO_3^{2-}$, $OH^-$, $SiO_3^{2-}$, x vaut 1, y désigne 1 ou 2, m et n désignent indépendamment l'un de l'autre 1 ou 2 avec n.y=m.x.

A titre de base minérale utilisable selon l'invention on peut citer, le carbonate de sodium, le carbonate de potassium, la soude, la potasse, le métasilicate de sodium, le métasilicate de potassium. De préférence la base minérale est un carbonate alcalin.

De manière avantageuse, la composition (B) présente une teneur en base(s) minérale(s) allant de 0,01 à 30 % en poids, de préférence de 0,1 à 20 % en poids par rapport au poids de ladite composition.

De préférence, le rapport pondéral amine(s) organique(s) dont le $pK_b$ est inférieur à 12 à 25°C / base(s) minérale(s) va de 0,1 à 10.

La composition cosmétique (B) peut être une composition anhydre ou aqueuse. Par composition aqueuse, on entend une composition comprenant plus de 5 % en poids d'eau, de préférence plus de 10 % en poids d'eau, et de manière encore plus avantageuse plus de 20 % en poids d'eau.

De préférence, la composition cosmétique (B) est une composition aqueuse.

De préférence la composition (B) contient de l'eau. Encore plus préférentiellement la concentration en eau peut aller de 10 à 90 %, mieux de 20 à 80 % du poids total de la composition.

La composition peut éventuellement comprendre un ou plusieurs solvants.

A titre de solvant organique, on peut par exemple citer, les alcanols, linéaires ou ramifiés, en $C_2$-$C_4$, tels que l'éthanol et l'isopropanol ; le glycérol ; les polyols et éthers de polyols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Le ou les solvants, s'ils sont présents, représentent une teneur allant habituellement de 1 à 40 % en poids par rapport au poids de la composition cosmétique (B), et de préférence allant de 5 à 30 % en poids.

La composition cosmétique (B) peut également comprendre des additifs classiques tels que ceux qui ont été listés auparavant et l'on pourra s'y reporter.

Le pH de la composition cosmétique (B) si elle est aqueuse est compris entre 2 et 13, de préférence entre 8 et 12. Le pH est adapté en utilisant des agents acidifiants ou alcalinisants additionnels, tels que ceux mentionnés ci-après.

Parmi les agents acidifiants additionnels, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

En ce qui concerne l'agent alcalinisant additionnel, s'il est présent, il peut être choisi parmi les amines organiques non salifiées décrites auparavant, ou éventuellement, bien que non préférentiellement, l'ammoniaque. Si toutefois l'ammoniaque était employé en tant qu'agent alcalinisant additionnel dans la composition (B), alors de préférence, sa teneur serait de préférence inférieure ou égale à 0,03% en poids de la composition finale (exprimée en $NH_3$), plus particulièrement inférieure ou égale à 0,01% en poids par rapport à la composition finale. Il est indiqué que la composition finale résulte du mélange des compositions (A), (B) et (C) ; ces mélanges étant réalisés soit avant application sur les fibres kératiniques (préparation extemporanée), soit directement sur les fibres kératiniques (applications successives avec ou sans prémélanges et sans rinçage intermédiaire).

De préférence, si la composition comprend de l'ammoniaque ou un de ses sels, alors la quantité d'agent(s) alcalinisant(s) est supérieure à celle de l'ammoniaque (exprimée en $NH_3$).

Enfin, le procédé est mis en oeuvre avec une composition (C) comprenant un ou plusieurs agents oxydants.

Plus particulièrement, le ou les agents oxydants sont choisis parmi le peroxyde d'hydrogène, le peroxyde d'urée, les bromates ou ferricyanures de métaux alcalins, les sels peroxygénés comme par exemple les persulfates, les perborates, les peracides et leurs précurseurs, les percarbonates de métaux alcalins ou alcalino-terreux et les peracides et leurs précurseurs.

On peut également utiliser à titre d'agent oxydant une ou plusieurs enzymes d'oxydo-réduction telles que les laccases, les peroxydases et les oxydoréductases à 2 électrons (telles que l'uricase), éventuellement en présence de leur donneur ou cofacteur respectif.

Cet agent oxydant est avantageusement constitué par du peroxyde d'hydrogène et notamment en solution aqueuse (eau oxygénée) dont la concentration peut varier, plus particulièrement de 0,1 à 50% en poids, et encore plus préférentiellement de 0,5 à 20% en poids, mieux de 1 à 15% en poids par rapport à la composition oxydante (C).

En fonction du degré d'éclaircissement souhaité, l'agent oxydant peut également comprendre un agent oxydant choisi de préférence parmi les sels peroxygénés.

La composition oxydante peut être aqueuse ou non. Par composition aqueuse, on entend une composition comprenant plus de 5 % en poids d'eau, de préférence plus de 10 % en poids d'eau, et de manière encore plus avantageuse plus de 20 % en poids d'eau.

De préférence, la composition (C) est une composition aqueuse.

Elle peut également comprendre un ou plusieurs solvants organiques.

A titre de solvant organique, on peut par exemple citer, les alcanols, linéaires ou ramifiés, en $C_2$-$C_4$, tels que l'éthanol et l'isopropanol ; le glycérol ; les polyols et éthers de polyols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Le ou les solvants, s'ils sont présents, représentent une teneur allant habituellement de 1 à 40 % en poids par rapport au poids de la composition oxydante (C), et de préférence de 5 à 30 % en poids.

La composition oxydante (C) peut comprendre un ou plusieurs agents acidifiants.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Habituellement, le pH de la composition oxydante (C), lorsqu'elle est aqueuse, est inférieur à 7.

La composition oxydante (C) peut également renfermer d'autres ingrédients classiquement employés dans le domaine, comme notamment ceux détaillés auparavant dans le cadre de la composition anhydre (A).

Enfin, la composition oxydante (C) se présente sous diverses formes, comme par exemple une solution, une émulsion ou un gel.

De préférence, la composition résultant du mélange des compositions (A), (B) et (C) est exempte d'ammoniaque.

Selon une variante, la composition obtenue après mélange des compositions (A), (B) et (C) décrites précédemment, est telle que, après mélange, la quantité de corps gras est supérieure à 20% en poids, de préférence supérieure à 25% en poids et de manière encore plus avantageuse, supérieure à 30% en poids.

Conformément à une première variante du procédé, on applique successivement et sans rinçage intermédiaire la composition résultant du mélange préalable à l'application des compositions (A) et (B) puis la composition oxydante (C).

Conformément à une deuxième variante du procédé, on applique sur les fibres kératiniques, sèches ou humides, une composition obtenue par mélange extemporané avant l'application, des compositions (A), (B) et (C). Cette variante est préférée.

Dans cette variante, les rapports pondéraux R1 des quantités de compositions (A) + (B) / (C) et R2 des quantités de compositions (A) / (B) varient de 0,1 à 10, et de préférence de 0,3 à 3.

En outre, indépendamment de la variante mise en oeuvre, le mélange présent sur les fibres (résultant soit du mélange extemporané des compositions (A), (B) et (C), soit de l'application successive de celles-ci) est laissé en place pour une durée, en général, de l'ordre de 1 minute à 1 heure, de préférence de 5 minutes à 30 minutes.

La température durant le procédé est classiquement comprise entre la température ambiante (entre 15 à 25°C)

et 80°C, de préférence entre la température ambiante et 60°C.

A l'issue du traitement, les fibres kératiniques humaines sont éventuellement rincées à l'eau, subissent éventuellement un lavage avec un shampooing suivi d'un rinçage à l'eau, avant d'être séchées ou laissées à sécher.

La présente invention est aussi relative à une composition anhydre comprenant un ou plusieurs corps gras, un ou plusieurs tensioactifs, une ou plusieurs amines organiques dont le $pK_b$ est inférieur à 12 à 25°C et une ou plusieurs bases minérales autres que l'ammoniaque.

De préférence, la composition anhydre comprend un ou plusieurs corps gras, un ou plusieurs tensioactifs, une ou plusieurs amines organiques dont le $pK_b$ est inférieur à 12 à 25°C et une ou plusieurs bases minérales choisies parmi le carbonate de sodium, le carbonate de potassium, la soude, la potasse, le métasilicate de sodium, le métasilicate de potassium.

De manière encore plus préférée, la ou les bases minérales utilisées dans la composition anhydre sont choisies parmi les carbonates alcalins.

Enfin, l'invention concerne un dispositif à plusieurs compartiments comprenant un premier compartiment renfermant la composition anhydre (A) comprenant un ou plusieurs corps gras et un ou plusieurs tensioactifs, un deuxième compartiment renfermant une composition cosmétique (B) comprenant une ou plusieurs amines organiques dont le $pK_b$ est inférieur à 12 à 25°C et une ou plusieurs bases minérales ainsi qu'éventuellement un ou plusieurs colorants d'oxydation et/ou un ou plusieurs colorants directs, et un troisième compartiment renfermant une composition (C) comprenant un ou plusieurs agents oxydants.

Les exemples suivants servent à illustrer l'invention sans toutefois présenter un caractère limitatif.

## EXEMPLES

### Procédé de coloration

Exemple 1

On prépare les compositions suivantes (quantités exprimées en grammes)

Composition anhydre A :

| | |
|---|---|
| Monolaurate de sorbitane oxyethyléné (4OE) | 21,7 |
| Silice pyrogénée à caractère hydrophobe | 11,1 |
| Huile de vaseline | Qsp 100 |

Composition cosmétique B :

| | |
|---|---|
| Carbonate de potassium | 7,25 |
| Monoéthanolamine | 9,50 |
| Para-phénylène diamine | 2,35 |
| Résorcinol | 2,37 |
| Métabisulfite de sodium en poudre | 0,70 |
| Acide erythorbique | 0,25 |
| Acide diéthylène triamine pentacetique, sel pentasodique en solution aqueuse à 40% | 1 |
| Ethanol | 8,80 |
| Propylène glycol | 6,20 |
| Hexylène glycol | 3,00 |
| Dipropylène glycol | 3,00 |

(suite)

| | |
|---|---|
| Eau déminéralisée | Qsp 100 |

Au moment de l'emploi, on mélange :

- 10 parties en poids de la composition (A),
- 4 parties en poids de la composition (B), et
- 15 parties en poids d'une composition aqueuse oxydante comprenant 6% en poids de peroxyde d'hydrogène à pH de 2,3 et comprenant environ 80 % d'eau.

Le mélange obtenu, dont le pH est environ 10, est ensuite appliqué sur une mèche de cheveux naturels à 90% blancs (BN) et sur une mèche de cheveux à 90% blancs permanentés (BP). Le rapport de bain « mélange/mèche » est respectivement de 10/1 (g/g).

Le temps de pause est de 30 minutes à 27°C. A l'issue de ce temps, les mèches sont rincées, puis lavées avec du shampooing Elsève multivitamines.

Résultats

La couleur des mèches a été évaluée dans le système CIE L* a* b*, au moyen d'un colorimètre Minolta Spectrophotomètre CM2600D.

a. Calcul de la montée ou variation de la couleur ($\Delta E_{ab}$*)

La montée de la coloration ($\Delta E_{ab}$*) a été évaluée dans le système CIE L* a* b*, au moyen d'un colorimètre Minolta Spectrophotomètre CM2600D. Dans ce système L* a* b*, L* représente l'intensité de la couleur, a* indique l'axe de couleur vert/rouge et b* l'axe de couleur bleu/jaune. Plus la valeur de L* est faible, plus la couleur est foncée ou très intense.

Dans le tableau ci-dessous, on calcule la valeur de $\Delta E_{ab}$* à partir des valeurs de L*a*b* selon l'équation suivante (i) :

$$\Delta E_{ab}* = \sqrt{(L*-L_o*)^2 + (a*-a_o*)^2 + (b*-b_o*)^2} \quad (i)$$

La montée de la coloration ($\Delta E_{ab}$*) a été calculée sur les mèches de cheveux blancs naturelles (BN) et sur les mèches de cheveux blancs permanentées.

Dans l'équation (i), pour les mèches de cheveux blancs naturels (BN), L*, a* et b* représentent les valeurs mesurées sur des mèches de cheveux blancs naturels après coloration, et $L_0$*, $a_0$* et $b_0$* représentent les valeurs mesurées sur des mèches de cheveux blancs naturels non colorés.

Dans l'équation (i), pour les mèches de cheveux blancs permanentés (BP), L*, a* et b* représentent les valeurs mesurées sur des mèches de cheveux blancs permanentés après coloration, et $L_0$*, $a_0$* et $b_0$* représentent les valeurs mesurées sur des mèches de cheveux blancs permanentés non colorés.

Plus la valeur de $\Delta E_{ab}$* est grande, meilleure est la montée ou la variation de la couleur.

b. Calcul de la sélectivité

On calcule également la valeur de ΔE (sélectivité) à partir des valeurs de L* a* b* mesurées selon l'équation suivante (ii) :

$$\Delta E = \sqrt{(L*-L_o*)^2 + (a*-a_o*)^2 + (b*-b_o*)^2} \quad (ii)$$

Dans l'équation (ii), L*, a* et b* représentent les valeurs mesurées sur des mèches de cheveux blancs naturels colorés, et $L_0$*, $a_0$* et $b_0$* représentent les valeurs mesurées sur des mèches de cheveux blancs permanentés colorés.

La sélectivité de la coloration ΔE correspond à la variation de la couleur entre des cheveux naturels, représentatifs de la nature des cheveux à la racine, et des cheveux permanentés qui sont représentatifs de la nature des cheveux à la pointe. Plus la valeur de ΔE est faible, plus la coloration est homogène entre la pointe et la racine des cheveux.

Les résultats sont donnés dans le tableau ci-dessous.

| | L* | a* | b* | $\Delta E_{ab}$* | ΔE Sélectivité |
|---|---|---|---|---|---|
| Mèche de cheveu naturelle non traitée | 58,0 | 0,3 | 12,2 | | |
| Mèche de cheveu permanentée non traitée | 61,8 | -0,01 | 13,3 | | |
| Mèche de cheveu naturelle traitée avec la composition selon l'invention | 19,2 | 2,1 | 5,2 | 39,5 | 1,3 |
| Mèche de cheveu permanentée traitée avec la composition selon l'invention | 18,3 | 1,8 | 4,3 | 44,5 | |

Comme le montre le tableau ci-dessus, on obtient une coloration puissante et peu sélective avec le procédé selon l'invention.

De plus, on n'observe aucune odeur agressive, ni pendant la préparation du mélange colorant, ni pendant le temps de pose sur mèches.

<u>Exemple 2</u>

On prépare les compositions suivantes (quantités exprimées en grammes)

<u>Composition anhydre A :</u>

| | |
|---|---|
| Monolaurate de sorbitane oxyethyléné (4OE) | 21,7 |
| Silice pyrogénée à caractère hydrophobe | 11,1 |
| Huile de vaseline | Qsp 100 |

<u>Composition cosmétique B :</u>

| | |
|---|---|
| Carbonate de potassium | 7,25 |
| Monoéthanolamine | 9,50 |
| Para-phénylène diamine | 2,39 |
| 4-amino-2-hydroxytoluène | 2,68 |
| Métabisulfite de sodium | 0,70 |
| Acide erythorbique | 0,25 |
| Acide diéthylène trimaine pentacetique, sel pentasodique en solution aqueuse à 40% | 1 |
| Ethanol | 8,80 |
| Propylène glycol | 6,20 |
| Hexylène glycol | 3,00 |
| Dipropylène glycol | 3,00 |
| Eau déminéralisée | Qsp 100 |

Au moment de l'emploi, on mélange :

- 10 parties en poids de la composition (A),
- 4 parties en poids de la composition (B), et
- 15 parties en poids d'une composition aqueuse oxydante comprenant 6% en poids de peroxyde d'hydrogène à pH de 2,3 et comprenant environ 80 % d'eau

Le mélange obtenu, dont le pH est environ 10, est ensuite appliqué sur une mèche de cheveux naturels à 90% blancs (BN) et sur une mèche de cheveux à 90% blancs permanentés (BP). Le rapport de bain « mélange/mèche » est respectivement de 10/1 (g/g).

Le temps de pause est de 30 minutes à 27°C. A l'issue de ce temps, les mèches sont rincées, puis lavées avec du shampooing Elsève multivitamines.

Résultats

La couleur des mèches a été évaluée dans le système CIE L* a* b*, au moyen d'un colorimètre Minolta Spectrophotomètre CM2600D.

a. Calcul de la montée ou variation de la couleur ($\Delta E_{ab}$*)

La montée de la coloration ($\Delta E_{ab}$*) a été évaluée dans le système CIE L* a* b*, au moyen d'un colorimètre Minolta Spectrophotomètre CM2600D selon l'équation (i) décrite ci-avant.

b. Calcul de la sélectivité

On calcule également la valeur de ΔE (sélectivité) à partir des valeurs de L* a* b* mesurées selon l'équation suivante (ii) décrite ci-avant.

Les résultats sont donnés dans le tableau ci-dessous.

| | L* | a* | b* | $\Delta E_{ab}$* | ΔE Sélectivité |
|---|---|---|---|---|---|
| Mèche de cheveu naturelle non traitée | 58,0 | 0,3 | 12,2 | | |
| Mèche de cheveu permanentée non traitée | 61,8 | -0,01 | 13,3 | | |
| Mèche de cheveu naturelle traitée avec la composition selon l'invention | 17,0 | 9,3 | -0,1 | 43,7 | 2,2 |
| Mèche de cheveu permanentée traitée avec la composition selon l'invention | 17,1 | 7,1 | 0,1 | 47,2 | |

Comme le montre le tableau ci-dessus, on obtient une couleur violette puissante et peu sélective avec le procédé selon l'invention.

De plus, on n'observe aucune odeur agressive, ni pendant la préparation du mélange colorant, ni pendant le temps de pose sur mèches.

**Procédé d'éclaircissement**

Exemple

On prépare les compositions suivantes (les quantités sont exprimées en grammes sauf indications contraires) :

Composition anhydre (A) :

| | |
|---|---|
| Monolaurate de sorbitane oxyethyléné (4OE) | 21,7 |
| Silice pyrogénée à caractère hydrophobe | 11,1 |
| Huile de vaseline | Qsp 100 |

Composition cosmétique (B) :

| | |
|---|---|
| Carbonate de potassium | 7,25 |
| Monoéthanolamine | 9,50 |
| Eau déminéralisée | Qsp 100 |

Au moment de l'emploi, on mélange :

- 10 parties en poids de la composition anhydre (A),
- 4 parties en poids de la composition cosmétique (B), et
- 15 parties en poids de composition aqueuse oxydante (C) comprenant 6 % de peroxyde d'hydrogène à pH de 2,3

Le mélange obtenu, dont le pH est d'environ de 9, est ensuite appliqué sur une mèche châtain (hauteur de ton 3) naturelle. Le rapport de bain « mélange/mèche » est respectivement de 10/1 (g/g).

Le temps de pose est de 30 minutes à 27°C. A l'issue de ce temps, les mèches sont rincées, puis lavées avec du shampooing Elsève multivitamines.

On obtient un bon niveau d'éclaircissement sans dégagement d'odeur agressive.

A titre de comparaison, on prépare la composition comparative 1 à base d'ammoniaque (sauf indication contraire, les quantités sont aussi exprimées en grammes).

Composition comparative 1 :

| | |
|---|---|
| Alcool oléique polyglycérolé à 2 moles de glycérol | 4 |
| Alcool oléique polyglycérolé à 4 moles de glycérol | 5,69 MA |
| Acide oléique | 3 |
| Amine oléïque à 2 moles d'oxyde d'éthylène vendue sous la dénomination commerciale ETHOMEEN 012 par la société Akzo | 7 |
| Laurylamino succinamate de diéthylaminopropyle, sel de sodium à 55% Matières active | 3,0 MA |
| Alcool oléïque | 5 |
| Diéthanolamide d'acide oléique | 12 |
| Alcool éthylique | 7 |
| Propylène glycol | 3,5 |
| Dipropylèneglycol | 0,5 |
| Monométhyléther de propyléneglycol | 9 |
| Acétate d'ammonium | 0,8 |
| Ammoniaque à 20% de $NH_3$ (dont 41,15% de $NH_4OH$) | 10,2 |
| Eau déminéralisée q.s.p. | 100 g |

Au moment de l'emploi, la composition comparative 1 est mélangée poids pour poids avec une composition oxydante aqueuse comprenant 6 % en poids de peroxyde d'hydrogène à pH de 2,3.

Le mélange ainsi obtenu, dont le pH est environ égal à 9, est ensuite appliqué sur une mèche châtain (hauteur de ton 3) naturelle. Le rapport de bain « mélange/mèche » est respectivement de 10/1 (g/g).

Le temps de pause est de 30 minutes à 27°C. A l'issue de ce temps de pause, la mèche est rincée, puis lavée avec du shampooing Elsève vitamines.

La mise en oeuvre du procédé selon l'invention ne dégage aucune odeur agressive, contrairement à la mise

en oeuvre du procédé avec la composition comparative 1 qui dégage une forte odeur d'ammoniaque.

## Revendications

1. Procédé d'éclaircissement ou de coloration des fibres kératiniques, en particulier des fibres kératiniques humaines telles que les cheveux, en présence d'un agent oxydant, **caractérisé en ce que** l'on applique sur lesdites fibres kératiniques :

   (a) une composition cosmétique anhydre (A) comprenant :

      (i) un ou plusieurs corps gras dans une teneur comprise entre 20 et 90% en poids par rapport au poids total de la composition et un ou plusieurs tensioactifs,

   (b) une composition cosmétique (B) comprenant une ou plusieurs amines organiques dont le $pK_b$ est inférieur à 12 et une ou plusieurs bases minérales, et
   (c) une composition (C) comprenant un ou plusieurs agents oxydants ;

   lorsque le procédé est un procédé de coloration des fibres kératiniques, la composition (B) comprend en outre un ou plusieurs colorants d'oxydation et/ou un ou plusieurs colorants directs ;
   dans lequel :

   i) on applique sur lesdites fibres kératiniques successivement et sans rinçage intermédiaire la composition résultant du mélange préalable à l'application des compositions (A) et (B) puis la composition oxydante (C), ou
   ii) on applique sur lesdites fibres kératiniques une composition obtenue par mélange extemporané avant l'application, des compositions (A), (B) et (C).

2. Procédé selon la revendication 1, **caractérisé en ce que** la composition cosmétique anhydre (A) présente une teneur en eau inférieure à 5% en poids, par rapport au poids total de la composition.

3. Procédé selon l'une quelconque des revendications précédentes, **caractérisé en ce que** le ou les corps gras sont choisis parmi les composés liquides ou pâteux à température ambiante et à pression atmosphérique.

4. Procédé selon l'une quelconque des revendications précédentes, **caractérisé en ce que** le ou les corps gras sont choisis parmi les alcanes inférieurs en $C_6$-$C_{16}$, les alcools gras, les esters d'acide gras, les esters d'alcool gras, huiles non siliconées d'origine minérale de plus de 16 atomes de carbone, ou d'origine végétale, ou synthétique, les cires non siliconées, les silicones.

5. Procédé selon l'une quelconque des revendications précédentes, **caractérisé en ce que** le ou les corps gras sont choisis parmi l'huile de vaseline, les polydécènes, les esters d'acides gras ou d'alcools gras, liquides ou leurs mélanges.

6. Procédé selon l'une quelconque des revendications précédentes, **caractérisé en ce que** la composition anhydre (A) comprend un ou plusieurs tensioactifs choisis parmi les tensioactifs non ioniques en particulier les tensioactifs non ioniques mono- ou poly- oxyalkylénés et mono- ou poly- glycérolés.

7. Procédé selon l'une quelconque des revendications précédentes, **caractérisé en ce que** l'amine organique présente un $pK_b$ inférieur à 10, de préférence inférieur à 6.

8. Procédé selon l'une quelconque des revendications précédentes, **caractérisé en ce que** la ou les amines organiques sont choisies parmi les alcanolamines, en particulier la monoéthanolamine, les acides aminés basiques, les composés comportant une fonction guanidine.

9. Procédé selon l'une quelconque des revendications précédentes, **caractérisé en ce que** la ou les bases minérales sont choisies parmi le carbonate de sodium, le carbonate de potassium, la soude, la potasse, le métasilicate de sodium, le métasilicate de potassium.

10. Procédé selon l'une quelconque des revendications précédentes, **caractérisé en ce que** la ou les bases minérales

sont choisies parmi les carbonates alcalins.

**11.** Procédé selon l'une quelconque des revendications précédentes **caractérisé en ce que** le rapport pondéral amine(s) organique(s) dont le $pK_b$ est inférieur à 12 à 25°C / base(s) minérale(s) va de 0,1 à 10.

**12.** Composition anhydre **caractérisée en ce qu'**elle comprend un ou plusieurs corps gras, un ou plusieurs tensioactifs, une ou plusieurs amines organiques dont le $pK_b$ est inférieur à 12 à 25°C, une ou plusieurs bases minérales autres que l'ammoniaque.

**13.** Dispositif à plusieurs compartiments comprenant un premier compartiment renfermant la composition anhydre (A) selon l'une quelconque des revendications 1 à 6, un deuxième compartiment renfermant une composition (B) selon l'une quelconque des revendications 1 et 7 à 11, et un troisième compartiment renfermant une composition (C) renfermant un ou plusieurs agents oxydants.

**Patentansprüche**

**1.** Verfahren zum Aufhellen oder Färben von Keratinfasern, insbesondere menschlichen Keratinfasern wie dem Haar, in Gegenwart eines Oxidationsmittels, **dadurch gekennzeichnet, dass** man auf die Keratinfasern:

(a) eine wasserfreie kosmetische Zusammensetzung (A), die

(i) eine oder mehrere Fettsubstanzen in einem Gehalt zwischen 20 und 90 Gew.-%, bezogen auf das Gesamtgewicht der Zusammensetzung, und ein oder mehrere Tenside umfasst,

(b) eine kosmetische Zusammensetzung (B), die ein oder mehrere organische Amine mit einem $pK_b$-Wert von weniger als 12 und eine oder mehrere anorganische Basen umfasst, und
(c) eine Zusammensetzung (C), die ein oder mehrere Oxidationsmittel umfasst,

aufbringt,
wobei dann, wenn es sich bei dem Verfahren um ein Verfahren zum Färben von Keratinfasern handelt, die Zusammensetzung (B) außerdem einen oder mehrere Oxidationsfarbstoffe und/oder einen oder mehrere Direktfarbstoffe umfasst;
wobei:

i) man auf die Keratinfasern aufeinanderfolgend und ohne Zwischenspülung die Zusammensetzung, die sich aus dem Mischen der Zusammensetzungen (A) und (B) vor dem Aufbringen ergibt, und dann die oxidierende Zusammensetzung (C) aufbringt oder
ii) man auf die Keratinfasern eine durch Mischen der Zusammensetzungen (A), (B) und (C) unmittelbar vor dem Aufbringen erhaltene Zusammensetzung aufbringt.

**2.** Verfahren nach Anspruch 1, **dadurch gekennzeichnet, dass** die wasserfreie kosmetische Zusammensetzung (A) einen Wassergehalt von weniger als 5 Gew.-%, bezogen auf das Gesamtgewicht der Zusammensetzung, aufweist.

**3.** Verfahren nach einem der vorhergehenden Ansprüche, **dadurch gekennzeichnet, dass** die Fettsubstanz bzw. die Fettsubstanzen aus Verbindungen, die bei Umgebungstemperatur und Normaldruck flüssig oder pastös sind, ausgewählt ist bzw. sind.

**4.** Verfahren nach einem der vorhergehenden Ansprüche, **dadurch gekennzeichnet, dass** die Fettsubstanz bzw. die Fettsubstanzen aus $C_6$-$C_{16}$-Niederalkanen, Fettalkoholen, Fettsäureestern, Fettalkoholestern, Nichtsilikonölen mineralischer Herkunft mit mehr als 16 Kohlenstoffatomen oder pflanzlicher oder synthetischer Herkunft, Nichtsilikonwachsen und Silikonen ausgewählt ist bzw. sind.

**5.** Verfahren nach einem der vorhergehenden Ansprüche, **dadurch gekennzeichnet, dass** die Fettsubstanz bzw. Fettsubstanzen aus Vaselineöl, Polydecenen, flüssigen Fettsäure- oder Fettalkoholestern oder Mischungen davon ausgewählt ist bzw. sind.

**6.** Verfahren nach einem der vorhergehenden Ansprüche, **dadurch gekennzeichnet, dass** die wasserfreie Zusammensetzung (A) ein oder mehrere Tenside, die aus nichtionischen Tensiden, insbesondere mono- oder polyoxyalkylenierten und mono- oder polyglycerinierten nichtionischen Tensiden, ausgewählt sind, umfasst.

7. Verfahren nach einem der vorhergehenden Ansprüche, **dadurch gekennzeichnet, dass** das organische Amin einen $pK_b$-Wert von weniger als 10 und vorzugsweise weniger als 6 aufweist.

8. Verfahren nach einem der vorhergehenden Ansprüche, **dadurch gekennzeichnet, dass** das organische Amin bzw. die organischen Amine aus Alkanolaminen, insbesondere Monoethanolamin, basischen Aminosäuren und Verbindungen mit einer Guanidinfunktion ausgewählt ist bzw. sind.

9. Verfahren nach einem der vorhergehenden Ansprüche, **dadurch gekennzeichnet, dass** die anorganische Base bzw. die anorganischen Basen aus Natriumcarbonat, Kaliumcarbonat, Natriumhydroxid, Kaliumhydroxid, Natriummetasilikat und Kaliummetasilikat ausgewählt ist bzw. sind.

10. Verfahren nach einem der vorhergehenden Ansprüche, **dadurch gekennzeichnet, dass** die organische Base bzw. die organischen Basen aus Alkalicarbonaten ausgewählt ist bzw. sind.

11. Verfahren nach einem der vorhergehenden Ansprüche, **dadurch gekennzeichnet, dass** das Gewichtsverhältnis von organischem Amin bzw. organischen Aminen mit einem $pK_b$-Wert von weniger als 12 bei 25°C zu anorganischer Base bzw. anorganischen Basen 0,1 bis 10 beträgt.

12. Wasserfreie Zusammensetzung, **dadurch gekennzeichnet, dass** sie eine oder mehrere Fettsubstanzen, ein oder mehrere Tenside, ein oder mehrere organische Amine mit einem $pK_b$-Wert von weniger als 12 bei 25°C und eine oder mehrere von wässrigem Ammoniak verschiedene anorganische Basen umfasst.

13. Vorrichtung mit mehreren Kompartimenten, die ein erstes Kompartiment, das die wasserfreie Zusammensetzung (A) nach einem der Ansprüche 1 bis 6 enthält, ein zweites Kompartiment, das eine Zusammensetzung (B) nach einem der Ansprüche 1 und 7 bis 11 enthält, und ein drittes Kompartiment, das eine Zusammensetzung (C), die ein oder mehrere Oxidationsmittel enthält, enthält, aufweist.

## Claims

1. Process for lightening or dyeing keratin fibres, in particular human keratin fibres such as the hair, in the presence of an oxidizing agent, **characterized in that** the following are applied to the said keratin fibres:

(a) an anhydrous cosmetic composition (A) comprising:

(i) one or more fatty substances in a content of between 20% and 90% by weight relative to the total weight of the composition and one or more surfactants,

(b) a cosmetic composition (B) comprising one or more organic amines with a $pK_b$ of less than 12 and one or more mineral bases, and
(c) a composition (C) comprising one or more oxidizing agents;

when the process is a process for dyeing keratin fibres, composition (B) also comprises one or more oxidation dyes and/or one or more direct dyes,
in which

i) the composition resulting from the mixing, prior to application, of compositions (A) and (B) and then the oxidizing composition (C) is applied successively and without intermediate rinsing to said keratin fibres, or
ii) a composition obtained by extemporaneous mixing, before application, of compositions (A), (B) and (C), is applied to said keratin fibres.

2. Process according to Claim 1, **characterized in that** the anhydrous cosmetic composition (A) has a water content of less than 5% by weight relative to the total weight of the composition.

3. Process according to either of the preceding claims, **characterized in that** the fatty substance(s) is (are) chosen

from compounds that are liquid or pasty at room temperature and at atmospheric pressure.

4. Process according to any one of the preceding claims, **characterized in that** the fatty substance(s) is (are) chosen from $C_6$-$C_{16}$ lower alkanes, fatty alcohols, fatty acid esters, fatty alcohol esters, non-silicone oils of mineral origin of more than 16 carbon atoms, or of plant or synthetic origin, non-silicone waxes, and silicones.

5. Process according to any one of the preceding claims, **characterized in that** the fatty substance(s) is (are) chosen from liquid petroleum jelly, polydecenes, and liquid esters of fatty acids or of fatty alcohols, or mixtures thereof

6. Process according to any one of the preceding claims, **characterized in that** the anhydrous composition (A) comprises one or more surfactants chosen from nonionic surfactants, in particular monooxyalkylenated or polyoxyalkylenated, and monoglycerolated or polyglycerolated nonionic surfactants.

7. Process according to any one of the preceding claims, **characterized in that** the organic amine has a $pK_b$ of less than 10 and preferably less than 6.

8. Process according to any one of the preceding claims, **characterized in that** the organic amine(s) is (are) chosen from alkanolamines, in particular monoethanolamine, basic amino acids, and compounds comprising a guanidine function.

9. Process according to any one of the preceding claims, **characterized in that** the mineral base(s) is (are) chosen from sodium carbonate, potassium carbonate, sodium hydroxide, potassium hydroxide, sodium metasilicate and potassium metasilicate.

10. Process according to any one of the preceding claims, **characterized in that** the mineral base(s) is (are) chosen from alkali metal carbonates.

11. Process according to any one of the preceding claims, **characterized in that** the organic amine(s) with a $pK_b$ of less than 12 at 25°C/mineral base(s) weight ratio ranges from 0.1 to 10.

12. Anhydrous composition, **characterized in that** it comprises one or more fatty substances, one or more surfactants, one or more organic amines with a $pK_b$ of less than 12 at 25°C and one or more mineral bases other than aqueous ammonia.

13. Multi-compartment device comprising a first compartment containing the anhydrous composition (A) according to any one of Claims 1 to 6, a second compartment containing a composition (B) according to any one of Claims 1 and 7 to 11, and a third compartment containing a composition (C) containing one or more oxidizing agents.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- GB 1026978 A
- GB 1153196 A
- FR 2801308
- DE 2359399
- JP 63169571 A
- JP 5063124 A
- EP 0770375 A
- WO 9615765 A
- DE 3843892
- DE 4133957
- WO 9408969 A
- WO 9408970 A
- FR 2733749 A
- DE 19543988
- FR 2886136 A
- WO 9515144 A
- WO 9501772 A
- EP 714954 A
- FR 2189006
- FR 2285851
- FR 2140205
- EP 1378544 A
- EP 1674073 A
- EP 1637566 A
- EP 1619221 A
- EP 1634926 A
- EP 1619220 A
- EP 1672033 A
- EP 1671954 A
- EP 1671955 A
- EP 1679312 A
- EP 1671951 A
- EP 167952 A
- EP 167971 A
- WO 06063866 A
- WO 06063867 A
- WO 06063868 A
- WO 06063869 A
- EP 1408919 A
- EP 1377264 A
- EP 1377262 A
- EP 1377261 A
- EP 1377263 A
- EP 1399425 A
- EP 1399117 A
- EP 1416909 A
- EP 1399116 A
- EP 1671560 A
- EP 1006153 A
- EP 1433472 A
- EP 1433474 A
- EP 1433471 A
- EP 1433473 A
- EP 6291333 A


### Littérature non-brevet citée dans la description


- **WALTER NOLL.** Chemistry and Technology of Silicones. Academie Press, 1968
- **TODD ; BYERS.** Volatile Silicone fluids for cosmetics. *Cosmetics and Toiletries,* vol. 91, 27-32